# FASCICULE DE BREVET EUROPEEN

(11) **EP 2 285 343 B1**
(45) Date de publication et mention de la délivrance du brevet: **27.08.2014**
(21) Numéro de dépôt: 09750023.5
(22) Date de dépôt: 21.04.2009
(51) Int. Cl.: A61K 8/97, A61Q 19/00, A61Q 19/08, A61K 36/815

(54) **UTILISATION D'UN EXTRAIT DE WOLFBERRY, POUR MAINTENIR ET/OU RESTAURER LA TONICITE ET/OU LA FERMETE DE LA PEAU**
VERWENDUNG EINES WOLFSBEERENEXTRAKTES ZUR BEWAHRUNG UND/ODER WIEDERHERSTELLUNG VON STRAFFER HAUT
USE OF A WOLFBERRY EXTRACT, FOR MAINTAINING AND/OR RESTORING THE TONUS AND/OR FIRMNESS OF THE SKIN

(30) Priorité: 21.04.2008 FR 0852680; 27.05.2008 US 56348 P
(43) Date de publication de la demande: 23.02.2011
(60) Demande divisionnaire: 14157702.3
(73) Titulaire: L'Oréal, 75008 Paris (FR); Nestec S.A., 1800 Vevey (CH)
(72) Inventeur: CASTIEL, Isabelle, F-06200 Nice (FR); GUENICHE, Audrey, F-92500 Rueil Malmaison (FR)
(74) Mandataire: Le Coupanec, Pascale A.M.P.
(86) Numéro de dépôt international: PCT/FR2009/050739
(87) Numéro de publication internationale: WO 2009/141542

(56) Documents cités:
- WO-A-01/45648
- WO-A-2005/092121
- KR-B1- 100 753 186
- US-A1- 2007 166 267
- ZHAO H. ET AL.: "Lycium barbarum glycoconjugates : effect on human skin and cultured dermal fibroblasts" PHYTOMEDICINE, vol. 12, 2005, pages 131-137, XP004956914
- DATABASE EMBASE ELSEVIER SCIENCE PUBLISHERS, AMSTERDAM, NL; J. Clin. Rehabilitative Tissue Eng. Res. 11(36) 9 septembre 2007 (2007-09-09), LIANG J. ET AL.: "Influence of Lycium barbarum polysaccharide on activity of superoxide dismutase in rat skins." XP002504582
- BENZIE I.F.F. ET AL.: "Enhanced bioavailabitlity of zeaxanthin in a milk-based formulation of wolfberry (Gou Qi Zi; Fructus barbarum L.)" BRITISH J. NUTRITION, vol. 96, 2006, pages 154-160, XP009072766
- PALOMBO P ET AL: "Beneficial long-term effects of combined oral/topical antioxidant treatment with the carotenoids lutein and zeaxanthin on human skin: A double-blind, placebo-controlled study", SKIN PHARMACOLOGY AND PHYSIOLOGY: JOURNAL OF PHARMACOLOGICAL ANDBIOPHYSICAL RESEARCH, S. KARGER AG, BASEL, CH, vol. 20, no. 4, 1 June 2007 (2007-06-01), pages 199-210, XP008160907, ISSN: 1660-5527, DOI: 10.1159/000101807

## Description

La présente invention se rapporte principalement à la prévention et/ou au traitement des peaux qualifiées de peaux relâchées.

La peau humaine est constituée par trois compartiments, à savoir un compartiment superficiel, l'épiderme, le derme et un compartiment profond, l'hypoderme.

Le derme fournit à l'épiderme un support solide. C'est également son élément nourricier. Il est principalement constitué de fibroblastes et d'une matrice extracellulaire composée elle-même principalement de collagène, d'élastine et d'une substance dite substance fondamentale, composants synthétisés par les fibroblastes.

La matrice extracellulaire (MEC) de la peau humaine est constituée de diverses macromolécules responsables de la résistance mécanique de la peau, de sa souplesse, de sa tonicité et de son élasticité, ainsi que des fonctions physiologiquement importantes (hydratation, thermorégulation et régulation de la perméabilité de la peau).

Les macromolécules de la MEC ont été arbitrairement classées en quatre familles. Les deux premières sont constituées de macromolécules fibreuses et structurales, les collagènes et l'élastine, alors que les deux autres sont des glycoconjugués (glycoprotéines et protéoglycanes).

Les collagènes représentent 70 % des protéines de la MEC. De nombreux types de collagène constituent la MEC, dont notamment les collagènes interstitiels (de type I, II, III) de structure fibrillaire, produits essentiellement par les fibroblastes, et responsables de la cohésion, de la rigidité et de la résistance mécanique, les collagène des lames basales (de type IV) synthétisés par les cellules adjacentes et dans la peau par les kératinocytes et jouant notamment un rôle mécanique, les collagènes formant des fibrilles d'ancrage de la membrane basale (lien derme-épiderme) exprimées par les kératinocytes épidermiques (de type VII) ou encore les collagènes de type V et XII, non fibrillaires, qui ne sont pas retrouvées au niveau cutané.

En particulier, le collagène de type IV est spécifique de la membrane basale et les collagènes de type VI et VII (collagènes microfibrillaires) sont retrouvés au niveau de la jonction dermo-épidermique.

La fermeté et la résistance à la pression de la peau, et notamment sa tonicité, dépendent surtout des fibres de collagène de la membrane basale et de la jonction dermo-épidermique.

Plus précisément, les fibres de collagène sont constituées de fibrilles scellées les unes aux autres, formant ainsi plus de dix types de structures différentes. La solidité du derme est en grande partie due à l'enchevêtrement des fibres de collagène tassées les unes contre les autres en tout sens. Les fibres de collagène participent ainsi à l'élasticité et à la tonicité de la peau et/ou des muqueuses.

Naturellement, les fibres de collagène sont constamment renouvelées mais ce renouvellement diminue avec l'âge ce qui entraîne un amincissement du derme. Cet amincissement du derme peut être également dû à des causes pathologiques, comme par exemple l'hypersécrétion d'hormones corticoïdes, certaines pathologies ou encore des carences vitaminiques.

De même, il est également admis que des facteurs extrinsèques comme les rayons ultraviolets, le tabac ou certains traitements (glucocorticoïdes, vitamine D et dérivés par exemple) ont également un effet sur la peau et son taux de collagène.

Par ailleurs, les fibres de collagène sont sensibles à certaines enzymes appelées collagénases. Les collagénases font partie d'une famille d'enzymes appelées métalloprotéinases qui sont elles-mêmes les membres d'une famille d'enzymes protéolytiques (endoprotéases ou endopeptidases). Leur surexpression chez l'homme et leur activation est liée à de nombreux processus, parfois pathologiques, qui impliquent la destruction et le remodelage de la matrice. L'exposition prolongée aux rayonnements ultraviolets, particulièrement aux rayonnements de type A et B, a ainsi pour effet une stimulation de l'expression des collagénases, particulièrement de la MMP1, constituant une des composantes du vieillissement cutanée photo-induit.

Par ailleurs, à la ménopause, les principales modifications concernant le derme sont une diminution du taux de collagène et de l'épaisseur dermique. Cela entraîne chez la femme ménopausée un relâchement de la peau et/ou des muqueuses du fait que la peau présente une élasticité diminuée.

D'autre part, chez les sujets en surpoids, et plus particulièrement lors d'une prise de poids, les adipocytes ont tendance à augmenter rapidement de volume (du fait d'un stockage de quantités croissantes de lipides). La forte pression exercée par les adipocytes sur le derme provoque rapidement une déformation de la surface de la peau. A terme, les fibres dégénèrent et la peau perd ses structures fondamentales, ce qui se traduit par une altération de ses propriétés viscoélastiques ou biomécaniques (perte de fermeté, de tonicité, d'élasticité).

En revanche, lors d'une perte de poids, et notamment au cours des régimes amincissants, le destockage rapide des adipocytes entraîne une diminution importante de la tension exercée par l'hypoderme sur les tissus de soutien. Par conséquent, le derme n'étant plus sous tension, le tissu conjonctif perd progressivement de sa cohésion : perte d'attache des fibroblastes au collagène, diminution de la quantité de néocollagène, distension des fibres d'élastine, dépolymérisation des protéoglycanes.

En conséquence, il ressort de ce qui précède que de nombreux facteurs entraînent ainsi la dégradation des fibres de collagène, avec toutes les conséquences que l'on peut envisager sur la structure, la tonicité et/ou la fermeté de la peau et/ou des muqueuses.

On comprend alors l'importance du collagène et des glycosaminoglycanes dans la structure des tissus, particulièrement de la peau et/ou des muqueuses, et l'importance qu'il y a à combattre sa dégradation pour ainsi lutter contre le vieillissement, qu'il soit chronologique ou photoinduit, et ses conséquences, notamment sur l'amincissement du derme et/ou la dégradation des fibres de collagène, cette dernière conséquence entraînant en particulier l'apparence de peau molle, contre laquelle l'objet de la présente invention est précisément de lutter.

Ainsi, la présente invention vise à proposer l'utilisation cosmétique d'une quantité efficace d'un extrait de Wolfberry, comme agent pour maintenir et/ou restaurer la tonicité et/ou la fermeté de la peau.

De manière inattendue, les inventeurs ont en effet constaté qu'un tel extrait de Wolfberry, manifeste avantageusement une activité bénéfique de restructuration de la peau.

Le terme Wolfberry (également connu sous le nom Goji, ou encore sous les dénominations Chinese boxthorn ou Matrimony Vine) désigne habituellement différentes espèces du genre *Lycium,* de la famille des Solanaceae.

Parmi les différentes espèces du genre *Lycium,* le *Lycium barbarum* et le *Lycium chinense* sont les plus communément utilisés dans la médecine orientale.

Par exemple, le fruit du *Lycium barbarum,* également appelé *Fructus lycii,* est connu depuis de nombreuses années, tout particulièrement en Asie, à titre de plante médicinale aidant à se maintenir, de façon générale, en bonne santé.

Des travaux récents ont par ailleurs mis en évidence l'effet du *Lycium barbarum* sur l'amélioration et la régulation du système immunitaire, ainsi que ses propriétés anti-âge et de prévention du cancer, ses propriétés de diminution des taux de lipides et de sucre dans le sang, ou encore de diminution de la pression artérielle.

Le document US 2006/198810 enseigne par ailleurs l'efficacité d'une association d'un extrait de Wolfberry avec notamment des fruits du rosier et un extrait d'Angélique chinoise sur la stimulation de la synthèse des lipides dans les cellules cutanées.

Le document US 2007/0166267 concerne quant à lui des compositions à usage topique associant un extrait de *Lycium barbarum* et des oligopeptides acylés, et leur utilisation notamment pour améliorer la matrice fibroblastique.

Les propriétés de certains constituants du *Lycium barbarum* sont également déjà connues. Ainsi, le dipalmitate de zéaxanthine est connu pour ses propriétés anti-oxydantes et anti-inflammatoires, tandis que le polysaccharide de *Lycium barbarum* (LBP) et l'acide 2-O-(β-D-glucopyranosyl) ascorbique (analogue de vitamine C) sont quant à eux déjà connus pour leurs propriétés immuno-stimulantes et anti-oxydantes.

Contre toute attente, les inventeurs ont désormais découvert qu'un extrait de Wolfberry, s'avère posséder un effet bénéfique au niveau de la tonicité de la peau. Son administration raffermit la peau, et prévient efficacement son relâchement.

Les inventeurs ont notamment découvert qu'un tel extrait de Wolfberry, permet d'augmenter l'expression du collagène IV.

Ils ont également découvert qu'un tel extrait de Wolfberry, peut permettre d'inhiber l'expression de la gélatinase et celle de la métalloprotéinase de type 2 de la matrice extracellulaire.

La présente invention concerne ainsi l'utilisation cosmétique par voie orale d'une quantité efficace d'un extrait de Wolfberry obtenu par extraction de fruit de Wolfberry d'au moins un végétal de l'espèce *Lycium barbarum* dans un milieu liquide contenant du lait ou des protéines de lait comme agent pour maintenir et/ou restaurer la tonicité et/ou la fermeté de la peau en vue de prévenir et/ou traiter les peaux relâchées, et notamment comme agent raffermissant de la peau.

Au sens de la présente invention, l'expression « extrait de Wolfberry » désigne indifféremment un ou plusieurs composés. Il s'agit plus particulièrement d'un mélange de composé(s) existant naturellement dans un des matériaux végétaux (fruits, feuilles...) formant le Wolfberry, et plus particulièrement de son fruit, et qui en a été isolé généralement par extraction.

Selon un mode de réalisation l'extrait de Wolfberry, peut être destiné à atténuer l'aspect de la cellulite et de la peau d'orange.

Selon un autre mode de réalisation considéré selon l'invention, l'extrait de Wolfberry, peut être destiné à traiter les vergetures ou prévenir leur apparition.

Selon un mode de réalisation, l'extrait de Wolfberry, peut être destiné à traiter les atteintes cutanées liées à la ménopause.

Les vergetures se présentent comme des cicatrices en forme de stries légèrement creusées de coloration rosée ou nacrée. Elles se localisent principalement aux endroits où la peau est étirée : ventre, hanches, cuisses, fesses, seins.

Selon un mode de réalisation, l'extrait de Wolfberry, peut être destiné à augmenter l'expression du collagène IV.

Selon un autre mode de réalisation, l'extrait de Wolfberry, peut être destiné à inhiber l'expression de la gélatinase.

Selon encore un autre mode de réalisation, l'extrait de Wolfberry, peut être destiné à inhiber l'expression de la métalloprotéinase de type 2 de la matrice extracellulaire.

L'invention concerne également un procédé cosmétique de traitement et/ou de prévention des peaux relâchées comprenant l'administration par voie orale à un sujet d'une quantité efficace d'un extrait de Wolfberry obtenu par extraction de fruit de Wolfberry d'au moins un végétal de l'espèce *Lycium barbarum* dans un milieu liquide contenant du lait ou des protéines de lait.

Selon un mode de réalisation, ledit sujet peut être une femme enceinte.

Selon un autre mode de réalisation, ledit sujet peut être un sujet suivant un régime amincissant.

La voie orale, qui est la voie d'administration préférée selon l'invention, est avantageuse à plusieurs titres. D'une part, son administration est immédiate et ne soulève aucune contrainte au sujet considéré. Par ailleurs, elle a un spectre d'efficacité global, c'est-à-dire à la fois sur l'ensemble de la peau et dans ses couches profondes.

Par « quantité efficace », on entend au sens de la présente invention une quantité suffisante pour obtenir l'effet attendu.

Au sens de la présente invention, le terme « prévenir » signifie réduire le risque de manifestation du phénomène considéré.

Au sens de la présente invention, le terme « traiter » signifie suppléer à un dysfonctionnement physiologique et plus généralement à diminuer, voire supprimer un désordre indésirable et dont la manifestation est notamment une conséquence de ce dysfonctionnement.

### Extrait de Wolfberry

Au sens de l'invention, le terme « Wolfberry » entend désigner les espèces du genre *Lycium* choisies parmi *Lycium barbarum* et *Lycium chinense,* et de préférence l'espèce *Lycium barbarum.*

L'extrait de Wolfberry considéré selon l'invention contient au moins de la zéaxanthine ou l'un de ses dérivés et/ou du polysaccharide de *Lycium barbarum* (LBP).

Le polysaccharide de *Lycium barbarum* (LBP) est un groupe de polysaccharides hydrosolubles de types protéines à arabinogalactane (AGP).

A titre de dérivé de la zéaxanhine, on peut notamment citer les esters et les diesters de zéaxanthine, et plus particulièrement le dipalmitate de zéaxanthine.

Ainsi, le fruit Wolfberry comprend naturellement une teneur en zéaxanthine (incluant le dipalmitate de zéaxanthine et autres dérivés de zéaxanthine) allant de 20 à 300 mg, et de préférence de 50 à 250 mg en poids, en particulier de 100 à 200 mg en poids pour 100 g de fruit.

Le fruit Wolfberry contient en outre de la vitamine C ou l'un de ses analogues.

A titre d'analogue de la vitamine C, on peut notamment citer le glucoside d'ascorbyle, et par exemple l'acide 2-O-(β-D-glucopyranosyl)ascorbique.

Selon un mode de réalisation, l'extrait de Wolfberry mis en oeuvre selon l'invention contient au moins du dipalmitate de zéaxanthine et du polysaccharide de *Lycium barbarum.*

Selon un mode de réalisation, l'extrait de Wolfberry mis en oeuvre selon l'invention contient au moins du dipalmitate de zéaxanthine, du polysaccharide de *Lycium barbarum* (LBP) et de l'acide 2-O-(β-D-glucopyranosyl)ascorbique.

En particulier, cet extrait peut avantageusement contenir jusqu'à 0,30 % en poids, par exemple de 0,01 à 0,30 % en poids, en particulier de 0,03 à 0,12 % en poids, voire de 0,06 à 0,10 % en poids de zéaxanthine ou dérivés incluant le dipalmitate de zéaxanthine et autres dérivés de zéaxanthine par rapport à son poids total.

L'extrait de Wolfberry peut être un extrait préparé à partir de tout matériel végétal issu de Wolfberry, ledit matériel ayant été obtenu par culture *in vitro* ou *in vivo.*

De préférence on utilise un extrait obtenu à partir du fruit de Wolfberry.

Plus particulièrement selon l'invention on utilise le fruit de l'espèce *Lycium barbarum.*

Toute méthode d'extraction connue de l'homme du métier peut être utilisée selon l'invention. On peut ainsi notamment utiliser à titre de solvant d'extraction un milieu lacté tel que décrit ci-après.

Selon un mode de réalisation préféré de l'invention, on utilise un extrait susceptible d'être préparé selon l'un des procédés décrits dans le document WO 2005/092121.

On peut ainsi utiliser un extrait de Wolfberry obtenu par extraction de fruit de Wolfberry d'au moins un végétal de l'espèce *Lycium barbarum* dans un milieu liquide lacté, c'est-à-dire contenant du lait ou des protéines du lait.

Selon ce mode de réalisation, l'extrait de Wolfberry peut notamment être obtenu par un procédé de préparation comprenant les étapes consistant à mélanger et broyer le fruit entier de Wolfberry dans un milieu liquide contenant du lait ou des protéines de lait, séparer les fibres insolubles pour obtenir une suspension aqueuse, et dessécher la suspension ainsi isolée pour obtenir une poudre.

Le cas échéant, une étape de pasteurisation peut être réalisée sur la suspension aqueuse obtenue après séparation des fibres insolubles, et avant l'étape consistant à dessécher la suspension pour obtenir une poudre.

Les étapes de mélange, de broyage et de séparation des fibres insolubles peuvent être réalisées à température ambiante et à pression atmosphérique.

Un tel extrait peut encore être qualifié de Lactowolfberry.

Selon un mode de réalisation, l'extrait de Wolfberry peut se présenter sous la forme d'une poudre dispersible dans l'eau. Cette formulation permet en effet d'augmenter la biodisponibilité de la zéaxanthine de l'extrait de Wolfberry.

L'extrait de Wolfberry, et plus particulièrement du Lactowolfberry, est présent en une quantité efficace pour conférer aux compositions dans lesquelles il est formulé, les propriétés requises selon l'invention.

Ainsi, les compositions cosmétiques de l'invention peuvent comprendre une quantité en matière sèche d'extrait de Wolfberry allant de 0,5 à 20 % en poids par rapport au poids total de la composition le comprenant.

Pour des raisons manifestes, cette quantité en extrait de Wolfberry peut varier dans une large mesure et peut dépendre notamment de l'activité désirée.

Par exemple, l'extrait de Wolfberry, et plus particulièrement le Lactowolfberry peut être mis en oeuvre en une quantité en matière sèche allant de 0,5 % à 20 % en poids, et en particulier à raison d'au moins 5 % en poids, de préférence d'au moins 10 % en poids, par rapport au poids total de la composition orale selon l'invention le comprenant.

Selon une variante de réalisation de l'invention, l'extrait de Wolfberry, peut être avantageusement associé à au moins une quantité efficace d'au moins un microorganisme, notamment probiotique, de l'une de ses fractions ou de l'un de ses métabolites.

La combinaison d'un extrait de Wolfberry selon l'invention avec un tel microorganisme permet en effet de renforcer l'effet obtenu sur les peaux relâchées.

### Microorganismes, et notamment microorganismes probiotiques

Les microorganismes convenant à l'invention sont des microorganismes qui peuvent être administrés sans risques à l'animal ou l'homme.

En particulier, on utilise dans la présente invention au moins un microorganisme dit de type probiotique.

Au sens de la présente invention, on entend par « microorganisme probiotique », un microorganisme vivant qui, lorsqu'il est consommé en quantité adéquate, a un effet positif sur la santé de son hôte « joint FAO/WHO Expert Consultation on Evaluation of Health and Nutritional Properties of Probiotic in Food Including Powder Milk with Live Lactic Acid Bacteria, 6 octobre 2001 », et qui peut en particulier améliorer l'équilibre microbien intestinal.

Ces microorganismes convenant à l'invention peuvent être choisis notamment parmi les ascomycètes telles que *Saccharomyces, Yarrowia, Kluyveromyces, Torulaspora, Schizosaccharomyces pombe, Debaromyces, Candida, Pichia, Aspergillus* et *Penicillium,* des bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus et Lactobacillus* et leurs mélanges.

Comme ascomycètes convenant tout particulièrement à la présente invention, on peut en particulier citer *Yarrowia lipolitica et Kluyveromyces lactis,* de même que *Saccharomyces cereviseae, Torulaspora, Schizosaccharamyces pombe, Candida* et *Pichia.*

Des exemples spécifiques de microorganismes probiotiques sont *Lactobacillus acidophilus, Lactobacillus alimentarius, Lactobacillus curvatus, Lactobacillus delbruckii subsp. Lactis, Lactobacillus gasseri, Lactobacillus johnsonii, Lactobacillus reuteri, Lactobacillus paracasei, Lactobacillus rhamnosus (Lactobacillus GG), Lactobacillus sake, Lactococcus lactis, Streptococcus thermophilus, Staphylococccus carnosus,* et *Staphylococcus xylosus et leurs mélanges.*

Plus particulièrement, il s'agit de microorganismes probiotiques issus du groupe des bactéries lactiques, comme notamment les *Lactobacillus.* A titre illustratif de ces bactéries lactiques, on peut plus particulièrement citer les *Lactobacillus johnsonii, Lactobacillus paracasei, Lactobacillus reuteri, Lactobacillus rhamnosus* et leurs mélanges.

Les espèces convenant tout particulièrement sont les *Lactobacillus johnsonii,* notamment la souche déposée suivant le traité de Budapest avec l'Institut Pasteur (28 rue du Docteur Roux, F-75024 Paris cedex 15) sous la désignation suivante CNCM I-1225, et plus particulièrement les *Lactobacillus paracasei* dont plus préférentiellement la souche déposée le 12 janvier 1999 sous la désignation CNCM I-2116, et leurs mélanges.

Selon un mode de réalisation, l'extrait de Wolfberry peut être associé à un microorganisme de l'espèce *Lactobacillus paracasei,* l'une de ses fractions ou l'un de ses métabolites, et de préférence la souche *Lactobacillus paracasei* déposée le 12/01/99 sous la désignation CNCM I-2116.

Le microorganisme peut être mis en oeuvre à raison de 0,00001 à 20 % en poids, en particulier de 0,001 à 20 % en poids et plus particulièrement de 0,01 à 10 % en poids par rapport au poids total d'une composition en contenant.

D'une manière générale, les compositions à application topique hors invention comprennent généralement de 0,0001 à 30 %, en particulier de 0,001 à 15 % et plus particulièrement de 0,1 à 10 % en un ou plusieurs microorganismes notamment probiotiques.

Ce ou ces microorganisme(s) peu(ven)t être inclus dans les compositions selon l'invention sous une forme vivante, semi-active ou inactivée, morte.

Il(s) peu(ven)t également être inclus sous forme de fractions de composants cellulaires ou sous la forme de métabolites. Le ou le(s) microorganisme(s), métabolite(s) ou fraction(s) peu(ven)t également être introduit(s) sous la forme d'une poudre, d'un liquide, d'un surnageant de culture ou l'une de ses fractions, dilué(e) ou non, ou encore concentré(e) ou non.

Dans le cas où les microorganismes sont formulés dans une composition sous une forme vivante, la quantité de microorganismes vivants peut varier de 10³ à 10¹⁵ ufc/g, en particulier de 10⁵ à 10¹⁵ ufc/g et plus particulièrement de 10⁷ à 10¹² ufc/g de microorganismes par gramme de composition.

L'extrait de Wolfberry, peut être formulé dans une composition qui peut se présenter sous toutes les formes galéniques normalement disponibles pour le mode d'administration oral.

Le support peut être de nature diverse selon le type de composition considérée.

En ce qui concerne plus particulièrement les compositions destinées à une administration par voie topique externe c'est-à-dire sur la peau, il peut s'agir de solutions aqueuses, hydroalcooliques ou huileuses, de dispersions du type des solutions ou dispersions du type lotion ou sérum, d'émulsions de consistance liquide ou semi-liquide du type lait, de suspensions ou émulsions, du type crème, de gel aqueux ou anhydre, de microémulsions, de microcapsules, de microparticules, ou de dispersions vésiculaires de type ionique et/ou non ionique.

Ces compositions sont préparées selon les méthodes usuelles.

Ces compositions peuvent notamment constituer des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des produits de maquillage comme des fonds de teint fluides, des laits de démaquillage, des laits corporels de protection ou de soin, des laits après-solaires, des lotions, gels ou mousses pour le soin de la peau, comme des lotions de nettoyage ou de désinfection, des lotions anti-solaires, des lotions de bronzage artificiel, des compositions pour le bain, des compositions déodorantes contenant un agent bactéricide, des gels ou lotions après-rasage, des crèmes épilatoires, ou des compositions contre les piqûres d'insectes.

Les compositions selon l'invention peuvent également consister en des préparations solides constituant des savons ou des pains de nettoyage.

Elles peuvent être également utilisées pour le cuir chevelu sous forme de solutions, de crèmes, de gels, d'émulsions, de mousses ou encore sous forme de compositions pour aérosol contenant également un agent propulseur sous pression.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique et/ou dermatologique. L'émulsionnant et le coémulsionnant peuvent être présents, dans la composition, en une proportion allant de 0,3 à 30 % en poids, et de préférence de 0,5 à 20 % en poids par rapport au poids total de la composition.

Lorsque la composition de l'invention est une solution ou un gel huileux, la phase grasse peut représenter plus de 90 % du poids total de la composition.

De façon connue, les formes galéniques dédiées à une administration topique peuvent contenir également des adjuvants habituels dans le domaine cosmétique, pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

Comme matières grasses utilisables dans l'invention, on peut citer les huiles minérales comme par exemple le polyisobutène hydrogéné et l'huile de vaseline, les huiles végétales comme par exemple une fraction liquide du beurre de karité, huile de tournesol et d'amandes d'abricot, les huiles animales comme par exemple le perhydrosqualène, les huiles de synthèse notamment l'huile de Purcellin, le myristate d'isopropyle et le palmitate d'éthyl hexyle, les acides gras insaturés et les huiles fluorées comme par exemple les perfluoropolyéthers. On peut aussi utiliser des alcools gras, des acides gras comme par exemple l'acide stéarique et comme par exemple des cires notamment de paraffine, carnauba et la cire d'abeilles. On peut aussi utiliser des composés siliconés comme les huiles siliconées et par exemple les cyclométhicone et diméthicone, les cires, les résines et les gommes siliconées.

Comme émulsionnants utilisables dans l'invention, on peut citer par exemple le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO^{®} par la société HENKEL, le mélange de PEG-6/PEG-32/Glycol Stéarate vendu sous la dénomination de Tefose^{®} 63 par société GATTEFOSSE, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (20OE).

Comme solvants utilisables dans l'invention, on peut citer les alcools inférieurs, notamment l'éthanol et l'isopropanol, le propylène glycol.

La composition de l'invention peut également contenir de façon avantageuse une eau thermale et/ou minérale, notamment choisie parmi l'eau de Vittel, les eaux du bassin de Vichy et l'eau de la Roche Posay.

Comme gélifiants hydrophiles, on peut citer les polymères carboxyliques tel que le carbomer, les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305^{®} par la société SEPPIC, les polysaccharides comme les dérivés cellulosiques tels que les hydroxyalkylcelluloses et en particulier les hydroxypropylcellulose et hydroxyéthylcellulose, les gommes naturelles telles que les guar, caroube et xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

Dans le cas d'une utilisation conforme à l'invention par voie orale, on privilégie l'utilisation d'un support ingérable.

Conviennent notamment comme supports alimentaires ou pharmaceutiques, le lait, le yaourt, le fromage, les laits fermentés, les produits fermentés à base de lait, des glaces, des produits à base de céréales fermentées, des poudres à base de lait, des formules pour enfants et nourrissons, des produits alimentaires de type confiserie, chocolat, céréales, des aliments pour animaux en particulier domestiques, des comprimés, gélules ou tablettes, des suppléments oraux sous forme sèche et les suppléments oraux sous forme liquide.

Pour l'ingestion, de nombreuses formes de réalisation de compositions orales et notamment de compléments alimentaires sont possibles. Leur formulation est réalisée par les procédés usuels pour produire des dragées, gélules, gels, émulsions, comprimés, capsules. En particulier, le(s) actif(s) selon l'invention peuvent être incorporés dans toute autre forme de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.

Selon un mode de réalisation particulier, les microorganismes annexes considérés selon l'invention peuvent être formulés au sein de compositions sous une forme encapsulée de manière à améliorer significativement leur durée de survie. Dans un tel cas, la présence d'une capsule peut en particulier retarder ou éviter la dégradation du microorganisme au niveau du tractus gastro-intestinal.

Bien entendu, les compositions topiques (hors invention) ou orales selon l'invention peuvent en outre contenir plusieurs autres actifs.

A titre d'actifs utilisables, on peut citer, les vitamines A, B3, B5, B6, B8, C, D, E, ou PP, les curcuminoïdes, les caroténoïdes, les composés polyphénols et minéraux, les sucres, les acides aminés, les catéchines, les OPC, les acides aminés soufrés et les acides gras polyinsaturés 3 et 6.

En particulier, on peut utiliser un complexe anti-oxydant comprenant les vitamines C et E, et au moins un caroténoïde, notamment un caroténoïde choisi parmi le β-carotène, le lycopène, l'astaxanthine, la zéaxanthine et la lutéine, des flavonoïdes telles que les catéchines, des proanthocyanidines, des anthocyanines, des ubiquinones, des extraites de café contenant des polyphénols et/ou des diterpènes, des extraits de chicorés, des extraits de ginkgo biloba, des extraits de raisins riches en proanthocyanidines, des extraits de piment, des extraits de soja, d'autres sources de flavonoïdes possédant des propriétés antioxydantes, des acides gras, des prébiotiques, de la taurine, du resveratrol, des acides aminés du sélénium, des précurseurs de glutathion.

Il peut également s'agir d'au moins un prébiotique ou un mélange de prébiotiques. Plus particulièrement, ces prébiotiques peuvent être choisis parmi les oligosaccharides, produits à partir du glucose, galactose, xylose, maltose, sucrose, lactose, amidon, xylane, l'hémicellulose, l'inuline, des gommes de type acacia par exemple, ou un de leurs mélanges. Plus particulièrement, l'oligosaccharide comprend au moins un fructo-oligosaccharide. Plus particulièrement, ce prébiotique peut comprendre un mélange de fructo-oligosaccharide et d'inuline.

Dans les formes galéniques topiques, on peut utiliser plus particulièrement comme actifs hydrophiles les protéines ou les hydrolysats de protéine, les acides aminés, les polyols notamment en C₂ à C₁₀ comme les glycérine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe Vera.

Pour ce qui est des actifs lipophiles, on peut utiliser le rétinol (vitamine A) et ses dérivés, le tocophérol (vitamine E) et ses dérivés, les céramides, les huiles essentielles et les insaponifiables (tocotriénol, sésamine, gamma oryzanol, phytostérols, squalènes, cires, terpènes).

Comme actifs susceptibles d'être plus particulièrement associés à une quantité efficace d'un extrait de Wolfberry, dans une formule galénique orale, on peut également considérer tous les ingrédients communément utilisés et/ou autorisés.

A titre illustratif, on peut citer les vitamines, les minéraux, les lipides essentiels, les oligoéléments, les polyphénols, les flavonoïdes, les phytoestrogènes, les antioxydants tels que l'acide lipoïque et le coenzyme Q10, les caroténoïdes, les prébiotiques, les protéines et les acides aminés, les mono et polysaccharides, les amino-sucres, les phytostérols et alcools triterpéniques d'origine végétale.

Il s'agit, en particulier, des vitamines A, C, D, E, PP et du groupe B. Parmi les caroténoïdes, on choisit de préférence, le béta-carotène, le lycopène, la lutéine, la zéazanthine et l'astaxanthine. Les minéraux et oligo-éléments particulièrement mis en oeuvre sont le zinc, le calcium, le magnésium, le cuivre, le fer, l'iode, le manganèse, le sélénium, le chrome (III). Parmi les polyphénols, on retient aussi en particulier les polyphénols de raisin, de thé, d'olive, de cacao, de café, de pomme, de myrtille, de sureau, de fraise, de canneberge, et d'oignon. De préférence parmi les phytoestrogènes, on retient les isoflavones sous la forme libre ou glycosylée, telles que la génistéine, la daidzéine, la glycitéine ou encore les lignanes, en particulier ceux du lin et du schizandra chinensis. Les acides aminés ou les peptides et les protéines les contenant, tels que la taurine, la thréonine, la cystéine, le tryptophane, la méthionine. Les lipides appartiennent de préférence au groupe des huiles contenant des acides gras mono et polyinsaturés tels que les acides oléique, linoléique, alpha-linolénique, gamma-linolénique, stearidonique, les acides gras oméga-3 de poisson à longue chaîne tels que l'EPA et le DHA, les acides gras conjugués issus de végétaux ou d'animaux tels que les CLA (Conjugated Linoleic Acid).

Ainsi, en particulier lorsque un extrait de Wolberry, considéré selon l'invention est destiné à une administration par voie orale, il peut être associé en outre à au moins un actif nutritionnel choisi parmi le lycopène, la vitamine C, la vitamine E et les composés polyphénols.

L'extrait de Wolberry, peut également être associé à d'autres actifs nutritionnels choisis parmi :
- les actifs nutritionnels anti-âge, tels que les antioxydants alimentaires, les nutriments aux propriétés anti-radicalaires et les cofacteurs des enzymes endogènes antioxydants, les vitamines A, C, E, les caroténoïdes, les xantophyles, les isoflavones, certains minéraux tels que le zinc, le cuivre, le magnésium, le sélénium, l'acide lipoïque, le co-enzyme Q10, la superoxyde dismutase (SOD) ou encore la taurine. Parmi les actifs anti-âges, on peut notamment citer les fractions insaponifiables extraits de lipides d'origine végétale, aloe vera, le collagène marin natif ou hydrolysé, les huiles végétales ou marines riches en acides gras oméga-3, en oméga-6 (y compris l'acide gamma-linolénique),
- les actifs nutritionnels photoprotection tels que : les antioxydants et les antiradicalaires : les vitamines A, C, E, caroténoïdes, xantophyles, certains minéraux tels que le zinc, le cuivre, le magnésium, le sélénium, la co-enzyme Q10, la superoxyde dismultase (SOD), les probiotiques,
- les ingrédients nutritionnels présentant des propriétés d'hydratation ou encore immunomodulatrices tels que l'extrait de polypodium leucotomos, les huiles végétales ou marines riches en acides gras oméga-3, en oméga-6, y compris l'acide gamma-linolénique,
- les actifs nutritionnels actifs sur les signes cliniques de la ménopause (par exemple bouffées de chaleur, ...), tels que les isoflavones, les lignanes, la DHEA, les extraits de yam, de sauge, de houblon, le calcium, le magnésium, les hydrolysats de protéines, les huiles végétales ou marines riches en acides gras oméga-3,
- les ingrédients nutritionnels mis en oeuvre dans le domaine de la minceur, tels que les extraits de thé vert, maté, marron d'inde, kola, caféine, théobromine, synéphrine, bromelaïne, éphédra, citrus aurantium, calcium, hoodia, garcinia, chitosan, fibres végétales (cactus, pommes, ananas, ...), fenouil, cassis, reine des près, radis noir.

Un procédé de traitement cosmétique peut être mis en oeuvre notamment en administrant des compositions cosmétiques et/ou dermatologiques telles que définies ci-dessus, selon la technique d'utilisation habituelle de ces compositions. Par exemple : applications de crèmes, de gels, de sérums, de lotions, de laits de démaquillage ou de compositions après-solaires sur la peau pour ce qui est de l'application topique.

Un procédé (hors invention) peut être ainsi mis en oeuvre par administration topique, journalière par exemple, de l'extrait de Wolfberry.

Un procédé de traitement cosmétique de prévention et/ou de traitement des peaux relâchées hors l'invention, et en particulier les procédés de traitement cosmétique destinés à atténuer l'aspect de la cellulite et de la peau d'orange, peuvent être mis en oeuvre notamment en appliquant les compositions cosmétiques et/ou dermatologiques ou associations telles que définies ci-dessus, selon la technique d'utilisation habituelle de ces compositions.

Dans le cadre d'une administration par voie topique (hors invention), il peut être avantageux d'effectuer en outre au moins un massage des zones de peau étirées, en particulier les zones de localisation des vergetures telles que décrites précédemment.

Selon un mode de réalisation, le procédé de traitement cosmétique selon l'invention peut être précédé d'un régime amincissant.

Un procédé cosmétique hors l'invention peut être mis en oeuvre par administration topique, journalière par exemple, de compositions cosmétiques et/ou dermatologiques, qui peut être par exemple formulée sous forme de gels, lotions, émulsions.

Le procédé selon l'invention peut comprendre une administration unique.

Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Dans la description et dans les exemples suivants, sauf indication contraire, les pourcentages sont des pourcentages en poids et les plages de valeurs libellées sous la forme « entre... et... » incluent les bornes inférieure et supérieure précisées.

Les ingrédients sont mélangés, avant leur mise en forme, dans l'ordre et dans des conditions facilement déterminées par l'homme de l'art.

Les exemples ci-après sont présentés à titre illustratif et non limitatif du domaine de l'invention.

L'extrait de Wolfberry mis en oeuvre dans les exemples figurant ci-après est préparé à partir des fruits de Wolfberry de la province de Ningxia en Chine. Les fruits entiers utilisés préférentiellement pour l'invention contiennent au moins 110 mg et préférentiellement 150 mg de zéaxanthine par rapport à 100g de fruit. Cet extrait peut être préparé selon l'un des procédés décrits dans l'exemple 1 ou 7 du document WO 2005/092121. Le Lactowolfberry ainsi obtenu contient environ 53 % de fruit de Wolfberry, 30 % de lait écrémé et 17 % de maltodextrine ou environ 64 % de fruit de Wolfberry et 36 % de lait écrémé.

L'extrait ainsi obtenu est mis en oeuvre dans les formulations précisées ci-après.

### FIGURES

Figure 1 : l'activité de l'activité de la métalloprotéinase de type 2 de la matrice extracellulaire ou matrix métalloprotéinase de type 2 (MMP2) ou aussi appelée gélatinase est déterminée sur des échantillons de peau de sujets âgés de plus de 65 ans, dont le régime alimentaire est supplémenté (barre grise) ou non (barre noire) à raison de 0,5 % de Lactowolfberry par jour pendant 44 jours.
Figure 2 : l'expression de la MMP-2 (gélatinase) est mesurée sur des échantillons de peau prélevés sur des sujets âgés de plus de 65 ans, dont le régime alimentaire est supplémenté (barre grise) ou non (barre noire) par 0,5 % de Lactowolfberry par jour pendant 44 jours.
Figure 3 : le taux d'expression du collagène IV est déterminé par immunohistochimie sur des coupes de peau prélevées sur des sujets âgés de plus de 65 ans, dont le régime alimentaire est supplémenté (traité) ou non (contrôle) par 0,5 % de Lactowolfberry par jour pendant 44 jours.

### Exemple 1 : Gel unidose

| | % pds |
|---|---|
| Lactowolfberry (correspondant à 0,008 % de zéaxanthine) | 10 |
| Lycopène | 10 |
| *Lactobacillus johnsonii* | 10¹⁰ cfu |
| Sirop de sucre | 50 |
| Maltodextrine | 17 |
| Gomme Xanthane | 0,8 |
| Benzoate de sodium | 0,2 |
| Eau | qsp 100 |

On peut prendre une dose de 200 à 400 ml par jour.

### Exemple 2 : Capsule

| | mg/capsule |
|---|---|
| Vitamine C | 60 |
| Lactowolfberry (correspondant à 10,66 µg de zéaxanthine) | 13,7 |
| Glycérine | 150 |
| Stéarate de magnésium | 0,02 |
| Arôme naturel | qs |

On peut prendre une à trois de ces capsules par jour.

### Exemple 3

On adjoint à la formulation de l'exemple 2 un complexe vitaminique comportant 60 mg de vitamine C, 100 µg de vitamine E et 6 mg de β-carotène.

### Exemple 4

On adjoint à la formulation de l'exemple 2 un complexe vitaminique comportant 100 mg de vitamine C, 100 µg de vitamine E et 6 mg de lycopène par capsule.

### Exemple de référence 5 : Lotion pour les corps sous forme de spray

| | (% en poids) |
|---|---|
| Lactowolfberry (correspondant à 0,004 % de zéaxanthine) | 5,00 |
| Antioxydant | 0,05 |
| Isopropanol | 40,00 |
| Conservateur | 0,35 |
| Eau | qsp 100,00 |

### Exemple de référence 6 : Crème raffermissante pour le soin du corps

| | (% en poids) |
|---|---|
| Lactowolfberry (correspondant à 0,004 % de zéaxanthine) | 5,00 |
| Antioxydant | 0,1 |
| Isopropanol | 40,00 |
| Stéarate de glycérol | 1,00 |
| Alcool cétylstéarylique/alcool cétylstéarylique oxyéthyléné à 33 moles OE (Sinnowax AO vendu par la société Henkel) | 3,00 |
| Alcool cétylique | 1,00 |
| Diméthicone (DC 200 Fluid vendu par la société Dow Corning) | 1,00 |
| Huile de vaseline | 6,00 |
| Myristate d'isopropyle (Estol IPM 1514 vendu par Unichema) | 3,00 |
| Glycérine | 20,00 |
| Conservateur | 0,30 |
| Eau | qsp 100,00 |

### Exemple de référence 7 : Gel pour le soin du corps

| | (% en poids) |
|---|---|
| Lactowolfberry (correspondant à 0,004 % de zéaxanthine) | 5,00 |
| Antioxydant | 0,1 |
| Vitamine C | 2,50 |
| Isopropanol | 40,00 |
| Conservateur | 0,30 |
| Eau | qsp 100,00 |

### Exemple 8 : Effet du Lactowolfberry sur la gélatinase (MMP-2)

L'étude a été réalisée sur des sujets âgés de plus de 65 ans soumis à un régime alimentaire comprenant ou non du Lactowolfberry obtenu comme indiqué précédemment (0,5 % de leur alimentation totale quotidienne) pendant 44 jours.

Des échantillons de peau ont été prélevés et analysés afin d'objectiver le marqueur cutané MMP-2.

### Exemple 8A : activité gélatinase

### Préparation des échantillons

Après élimination de la fraction hypodermique au scalpel du prélèvement cutané, 33 mg par ml de fragments de peau sont suspendus dans une solution de CaCl₂ à 10 mM contenant 0,25 % de Tween 20 et 2 mM de PMSF (mélange d'inhibiteurs enzymatiques).

Les échantillons sont broyés pendant deux minutes à 0 °C à l'aide d'un Ultraturrax IKA. Puis, l'extrait est centrifugé deux fois dix minutes à 10 000 x g.

Les mesures de l'activité gélatinase sont effectuées sur le surnageant, en duplicata, en utilisant le kit InnoZyme Gélatinase Assay Kit, Fluorogenic (Calbiochem).

Le taux de protéines totales contenues dans le surnageant est évalué par la méthode de LOWRY.

Les échantillons sont dilués au 1/2 dans le tampon d'activation du kit, préparé par dilution de la solution concentrée d'APMA (p-aminophenylmercuric acétate) au 1/1000 dans le tampon d'essai du kit (45µl de tampon d'activation + 45 µl d'échantillon).

Une gamme étalon est préparée à partir de la solution de travail du standard pro-MMP2 à 1µg/ml obtenue par dilution d'une solution mère à 20 µg/ml dans de l'eau, et dilutions successives. La gamme est comprise, au final, entre 0 et 600 ng/ml (en ng/ml : 0, 50, 100, 200, 300, 400, 500 et 600).

Une solution de travail du substrat de la MMP-2 est préparée par dilution au 1/5 de la solution du kit.

Le volume réactionnel final de 100 µl, comprenant 90 µl de standard ou d'échantillon ou 90 µl de tampon d'activation pour les blancs, et 10 µl de solution de travail de substrat est distribué dans les puits d'une microplaque.

La microplaque est laissée à incuber pendant 6h à 37 °C.

La fluorescence est mesurée à 405 nm après excitation à 320 nm.

### Résultats :

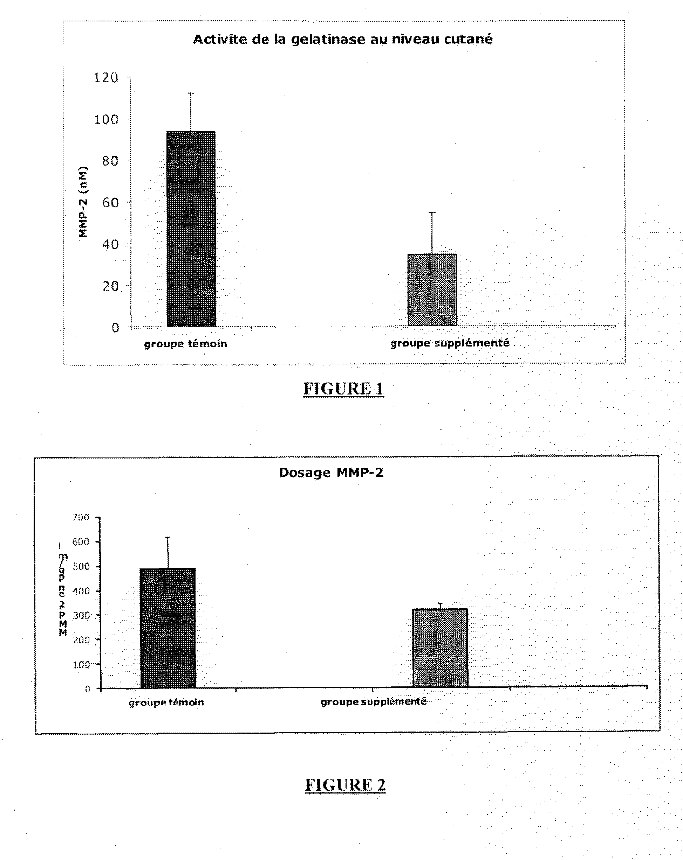

Les résultats (Fig. 1) montrent qu'un régime alimentaire supplémenté avec 0,5 % en Lactowolfberry par jour conduit à une diminution significative de l'activité de la gélatinase (MMP-2) au niveau cutané (significatif p < 0,05).

Le tableau ci-dessous résume les moyennes ± écartypes (ET) des activités MMP-2. Les résultats sont exprimés en nM.

| | moyennes | ET |
|---|---|---|
| Contrôle | 93,6 | 18,5 |
| Traité | 34,6 | 19,8 |

### Exemple 8B : dosage de la MMP-2

### Protocole opératoire

Le dosage de la MMP-2 (gélatinase) a été réalisé selon les instructions du fournisseur du kit Fluorokine MAP Human MMP-2 Kit (R&D Systems).

Deux témoins négatifs ont été réalisés pour chaque immunomarquage : un témoin non marqué pour vérifier l'autofluorescence des coupes et un témoin anticorps secondaire seul pour exclure toute fixation non spécifique de cet anticorps.

### Résultats

On observe une plus faible expression de la MMP-2 dans le groupe supplementé (traité) comparativement au groupe témoin (contrôle) (Fig. 2).

Le tableau ci-dessous résume les moyennes ± écartypes (ET) du dosage de la MMP-2. Les résultats sont exprimés en pg/ml.

| | moyennes | ET |
|---|---|---|
| Groupe témoin | 488,5 | 129,6 |
| Groupe supplementé | 318,2 | 25,6 |

### Exemple 9 : Effet du Lactowolfberry sur le collagène IV

L'étude a été réalisée sur des sujets âgés de plus de 65 ans soumis à un régime alimentaire comprenant ou non du Lactowolfberry obtenu comme indiqué précédemment (0,5 % de leur alimentation totale quotidienne) pendant 44 jours.

Des échantillons de peau ont été prélevés et analysés afin d'objectiver le marqueur cutané collagène IV.

### Protocole opératoire

L'immunomarquage du collagène IV est effectué par immunohistochimie avec un anticorps « antihuman collagen type IV polyclonal » [Rockland (revendu par Tebu-Bio)].

### Résultats

Le marquage collagène IV est localisé dans l'épiderme, exclusivement au niveau de la jonction dermo-épidermique (JDE) et dans le derme, au niveau des structures internes.

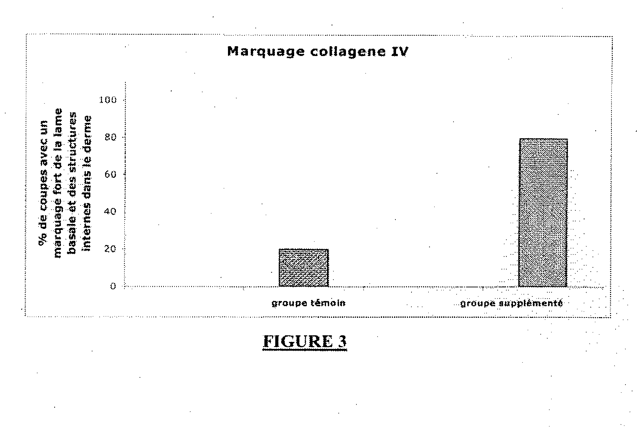

Une expression plus importante du collagène IV est observée dans le groupe supplémenté (traité) par rapport au témoin (contrôle) au niveau de la lame basale et des structures interne du derme (Fig. 3).

Le tableau ci-dessous résume le nombre de coupes effectuées dans chaque groupe où l'intensité du marquage collagène IV s'est avérée forte au niveau de la JDE:

| Groupes | Nombre de coupes avec un marquage moyen et fort de la JDE | Correspondance en % |
|---|---|---|
| témoin | 1/5 | 20 |
| supplementé | 4/5 | 80 |

Le Lactowolfberry permet d'obtenir une restructuration du réseau collagénique au niveau du tissu dermique et une augmentation de l'expression du collagène IV, l'un des marqueurs spécifiques de la jonction dermo-épidermique.

## Revendications

1. Utilisation cosmétique par voie orale d'une quantité efficace d'un extrait de Wolfberry obtenu par extraction de fruit de Wolfberry d'au moins un végétal de l'espèce *Lycium barbarum* dans un milieu liquide contenant du lait ou des protéines de lait comme agent pour maintenir et/ou restaurer la tonicité et/ou la fermeté de la peau en vue de prévenir et/ou traiter les peaux relâchées.

2. Utilisation selon la revendication 1, comme agent raffermissant de la peau.

3. Utilisation selon l'une quelconque des revendications précédentes, dans laquelle ledit extrait est destiné à atténuer l'aspect de la cellulite et de la peau d'orange.

4. Utilisation selon l'une quelconque des revendications précédentes, dans laquelle ledit extrait est destiné à traiter les vergetures ou prévenir leur apparition.

5. Utilisation selon l'une quelconque des revendications précédentes, dans laquelle ledit extrait est destiné à traiter les atteintes cutanées liées à la ménopause.

6. Utilisation selon l'une quelconque des revendications précédentes, dans laquelle ledit extrait est destiné à augmenter l'expression du collagène IV, et/ou à inhiber l'expression de la gélatinase, et/ou à inhiber l'expression de la métalloprotéinase du type 2 de la matrice extracellulaire.

7. Utilisation selon l'une quelconque des revendications précédentes, dans laquelle l'extrait de Wolfberry contient au moins de la zéaxanthine ou l'un de ses dérivés et/ou du polysaccharide de *Lycium barbarum* (LBP).

8. Utilisation selon l'une quelconque des revendications précédentes, dans laquelle l'extrait de Wolfberry contient de 0,01 % à 0,30 % en poids de zéaxanthine ou dérivés, incluant le dipalmitate de zéaxanthine et autres dérivés de zéaxanthine, par rapport à son poids total.

9. Utilisation selon l'une quelconque des revendications précédentes, dans laquelle l'extrait de Wolfberry contient au moins du dipalmitate de zéaxanthine et du polysaccharide de *Lycium barbarum.*

10. Utilisation selon l'une quelconque des revendications précédentes, **caractérisée en ce que** l'extrait de Wolfberry est associé à au moins une quantité efficace d'au moins un microorganisme probiotique, de l'une de ses fractions ou de l'un de ses métabolites.

11. Utilisation selon la revendication 10, **caractérisée en ce que** le microorganisme est l'espèce *Lactobacillus paracasei,* l'une de ses fractions ou l'un de ses métabolites.

12. Procédé cosmétique de traitement et/ou de prévention des peaux relâchées comprenant l'administration par voie orale à un sujet d'une quantité efficace d'un extrait de Wolfberry obtenu par extraction de fruit de Wolfberry d'au moins un végétal de l'espèce *Lycium barbarum* dans un milieu liquide contenant du lait ou des protéines de lait.

## Patentansprüche

1. Kosmetische orale Verwendung einer wirksamen Menge eines Wolfsbeerenfruchtextrakts, der durch Extraktion von Wolfsbeerenfrucht aus mindestens einer Pflanze der Art *Lycium barbarum* in einem flüssigen Medium erhalten wurde, das Milch oder Milchproteine enthält, als Mittel zum Aufrechterhalten und/oder Wiederherstellen der Spannkraft und/oder der Straffheit der Haut zur Verhinderung und/oder Behandlung schlaffer Häute.

2. Verwendung nach Anspruch 1 als Straffungsmittel für die Haut.

3. Verwendung nach einem der vorhergehenden Ansprüche, wobei der Extrakt dazu ausgelegt ist, das Aussehen der Cellulitis und der Orangenhaut zu mildern.

4. Verwendung nach einem der vorhergehenden Ansprüche, wobei der Extrakt dazu ausgelegt ist, Schwangerschaftsstreifen zu behandeln oder ihrem Auftreten zu verhindern.

5. Verwendung nach einem der vorhergehenden Ansprüche, wobei der Extrakt dazu ausgelegt ist, mit der Menopause einhergehende Hautschäden zu behandeln.

6. Verwerdung nach einem der vorhergehenden Ansprüche, wobei der Extrakt dazu ausgelegt ist, die Expression von Collagen IV zu steigern und/oder die Expression der Gelatinase zu hemmen, und/oder die Expression der Metalloproteinase des Typs 2 der extrazellulären Matrix zu hemmen.

7. Verwendung nach einem der vorhergehenden Absprüche, wobei der Wolfsbeerenextrakt mindestens Zeaxanthin oder eines seiner Derivate und/oder Polysaccharid von *Lycium barbarum* (LBP) enthält.

8. Verwendung nach einem der vorhergehenden Ansprüche, wobei der Wolfsbeerenextrakt 0,01 % bis 0,30 Gew.-% Zeaxanthin oder Derivate, einschließlich Zeaxanthindipalmitat und andere Zeaxanthinderivate, bezogen auf sein Gesamtgewicht enthält.

9. Verwendung nach einem der vorhergehenden Ansprüche, wobei der Wolfsbeerenextrakt mindestens Zeaxanthindipalmitat und Polysaccharid von *Lycium barbarum* enthält.

10. Verwendung nach einem der vorhergehenden Ansprüche, **dadurch gekennzeichnet, dass** der Wolfsbeerenextrakt mit mindestens einer wirksamen Menge von mindestens einem probiotischen Mikroorganismus, von einem seiner Bruchstücke oder von einem seiner Metaboliten kombiniert ist.

11. Verwendung nach Anspruch 10, **dadurch gekennzeichnet, dass** der Mikroorganismus die Art Lactobacillus paracasei, eines seiner Bruchstücke, oder einer seiner Metaboliten ist.

12. Kosmetisches Verfahren zur Behandlung und/oder Prävention schlaffer Häute, umfassend die orale Verabreichung an ein Individuum einer wirksamen Menge eines Wolfsbeerenextrakts, der durch Extraktion von Wolfsbeerenfrucht aus mindestens einer Pflanze der Art *Lycium barbarum* in einem flüssigen Medium erhalten wurde, das Milch oder Milchproteine enthält.

## Claims

1. Cosmetic use via oral administration of an effective quantity of an extract of Wolfberry obtained by extraction of the Wolfberry fruit of at least one plant of the species *Lycium barbarum* in a liquid medium containing milk or milk proteins as an agent for maintaining and / or restoring the tonicity and / or the firmness of the skin in view of preventing and/or treating sagging and loss of firmness of the skin.

2. Use according to claim 1 as a skin firming agent.

3. Use according to any one of the preceding claims, in which the said extract is administered to minimise the appearance of cellulite and reduce the "orange peel" effect on skin.

4. Use according to any one of the preceding claims, in which the said extract is administered for treating stretch marks or for preventing the appearance thereof.

5. Use according to any one of the preceding claims, in which the said extract is administered for treating skin disorders related to menopause.

6. Use according to any one of the preceding claims, in which the said extract is administered to bring about an increase in the expression of type IV collagen, and / or to inhibit the expression of gelatinase, and / or to inhibit the expression of (extracellular) matrix metalloproteinase type 2.

7. Use according to any one of the preceding claims, in which the said Woltberry extract contains at least zeaxanthin or one of the derivatives thereof and / or *Lycium barbarum* polysaccharide (LBP).

8. Use according to any one of the preceding claims, in which the said Woltberry extract contains 0.01% to 0.30% by weight of zeaxanthin or derivatives thereof, including zeaxanthin dipalmitate and other zeaxanthin derivatives, relative to its total weight.

9. Use according to any one of the preceding claims, in which the said Wolfberry extract contains at least zeaxanthin dipalmitate and *Lycium barbarum* polysaccharide.

10. Use according to any one of the preceding claims, **characterised in that** the wolfber extract is combined with at least an effective amount of at least one probiotic microorganism, one of the fractions thereof or one of the metabolites thereof.

11. Use according to claim 10, **characterised in that** the microorganism is of the species Lactobacillus paracasei, one of the fractions thereof or one of the metabolites thereof.

12. A method for the cosmetic treatment and / or prevention of sagging and loss of firmness of skin comprising of the oral administration to a subject of an effective quantity of a Wolfberry extract obtained by extraction of the Wolfberry fruit of at least one plant of the species *Lycium barbarum* in a liquid medium containing milk or milk proteins.
